# EUROPEAN PATENT SPECIFICATION

(11) **EP 2 623 057 B1**
(45) Date of publication and mention of the grant of the patent: **03.01.2024**
(21) Application number: 10857807.1
(22) Date of filing: 28.09.2010
(51) Int. Cl.: A61B 17/58, A61B 17/80

(54) **BONE PLATE AND BONE PLATE SYSTEM**
KNOCHENPLATTE UND KNOCHENPLATTENSYSTEM
LAME OSSEUSE ET SYSTÈME DE LAME OSSEUSE

(43) Date of publication of application: 07.08.2013
(73) Proprietor: Olympus Terumo Biomaterials Corp., Shinjuku-ku, Tokyo 163-0914 (JP)
(72) Inventor: MIZUNO, Hitoshi, Tokyo 163-0914 (JP); KURODA, Koichi, Tokyo 163-0914 (JP)
(74) Representative: Schicker, Silvia
(86) International application number: PCT/JP2010/066791
(87) International publication number: WO 2012/042592

(56) References cited:
- JP-A- 2007 151 674
- JP-A- 2007 506 450
- JP-A- 2007 515 990
- JP-A- 2010 022 866
- JP-A- 2010 522 019
- US-A1- 2005 165 400

## Description

### {Technical Field}

The present invention relates to a bone plate and a bone plate system.

### {Background Art}

As bone plates used for fixing part of a long bone, including fractured femur and tibia, there are conventionally known band-plate-shaped bone plates that have screw holes into which are tightened male threads provided on the heads of a plurality of screws to be tightened into the long bone (for example, see PTL 1).

Furthermore, there are known bone plates that have, in each screw hole, at least three screw-thread rows that are circumferentially segmented by at least three recesses (for example, see PTL 2).
JP 2010-022866 A discloses different examples of plates for osteosynthesis. In one embodiment, two threads are inclined at an angle with respect to a vertical axis.

### {Citation List}

### {Patent Literature}

{PTL 1} Japanese Translation of PCT International Application, Publication No. 2007-500069
{PTL 2} Japanese Translation of PCT International Application, Publication No. 2007-506450

### {Summary of Invention}

### {Technical Problem}

However, although PTL 1 proposes adoption of a fixing structure allowing selection of a screw fixing direction in order to improve the ease of handling of a bone plate, a specific structure having a sufficient strength as a bone plate to be used for a lower leg is not disclosed. Furthermore, in a plate that is also disclosed in PTL 1 and that does not allow selection of a screw fixing direction, there is a disadvantage in that the shape of the bone plate needs to be accurately deformed at an operation site.

In the bone plate shown in PTL 2, when the male thread provided on the head of a screw is tightened into the at least three screw-thread rows segmented by the recesses on a female thread, the axis of the screw can be inclined by an angle corresponding to one pitch by engaging a screw thread of the male thread with a different screw thread of the female thread. In this case, however, a reduction in strength due to a reduction in the area of portions where the male thread is engaged with the female thread, and the occurrence of metallic powder caused when the crests of screw threads and side surfaces of the screw threads are brought into contact with each other are issues of concern.

The present invention has been made in view of the above-described circumstances, and an object thereof is to provide a safe and ease-of-handle bone plate and bone plate system capable of tightening screws into a bone (in particular, a lower leg, a tibia) at desired angles while avoiding a troublesome bone-plate deforming task at an operation site and risks, such as the occurrence of metallic powder and implant fracture.

### {Solution to Problem}

In order to achieve the above-described object, the present invention provides a bone plate having the features of claim 1.

According to a first aspect, the present invention provides a bone plate configured to be formed into a band plate disposed along a bone surface, the bone plate comprising a plurality of screw holes penetrating in a plate thickness direction, wherein at least one of the screw holes that is located at an end in a longitudinal direction has a female thread. The female thread is formed by cutting a screw thread along an axis of a prepared hole and two or more axes inclined with respect to the axis of the prepared hole, each of the axes having a respective direction. Cutting is performed by tapping in the directions of the two or more inclined axes such that crests of the screw thread facing each other in the longitudinal direction are aligned with a crest shift therebetween, wherein the crest shift is selected from the group of 1/3, 0.5, 1 and 1.5 crests.

According to the bone plate of the invention, in the state in which the bone plate is disposed along the bone surface, screws are tightened into the bone through the screw holes, and, in a final phase of tightening, male threads provided on the screws are tightened into the female threads of the screw holes, provided in the bone plate, thereby fixing the screws to the bone plate, which makes it possible to fix the bone plate to the bone.

In this case, the male threads of the screws are each formed by cutting a screw thread along the axis of a prepared hole and at least one axis inclined with respect to this axis. In cutting the screw thread, the original screw thread is partially cut to form the screw thread of the female thread along the plurality of axes. Therefore, when the bone plate is fixed to the surface of the bone, it is possible to tighten the screw into the screw hole in a plurality of axis directions and to prevent a disadvantageous situation in which the female thread is stripped through tightening, thus producing cutting powder.

As a result, the angle of the screw fixed to the bone plate can be selected from a plurality of angles, and the screw can be fixed to the bone plate by selecting any appropriate angle.

Thus, as in high tibial osteotomy for knee osteoarthritis, even when the surface shape of a tibia that has been cut is different in each case, the practitioner need not deform the bone plate corresponding to the surface shape of the tibia each time at an operation site, and it is possible to select an appropriate fixing angle for the tibia and to tighten the screw into the bone plate. Furthermore, the screw hole need not be unnecessarily machined into an elliptical hole or an elongated hole.

Furthermore, even if the bone plate is deformed based on the judgment of a doctor, only a very small amount of deformation is required, which is not troublesome. Furthermore, the screw hole can be formed into a round shape having substantially the same size as a usual locking hole (screw hole that merely has a female thread formed along one axis). Therefore, unlike conventional bone plates with which it is possible to select the fixing angle of a screw, it is not necessary to enlarge a surrounding structure in order to ensure the strength, thus minimizing the invasiveness to the patient.

As the method of cutting a screw thread, tapping is be used. A tap used in tapping may be a tap receiving a tapered thread or a tap receiving a straight thread.

In the above-described first aspect, axes of the plurality of screw holes disposed adjacent to each other in the longitudinal direction may be located at mutually shifted positions or at parallel positions at intervals in a direction perpendicular to the longitudinal direction.

By doing so, the screws can be tightened such that the axes of the screws tightened into the screw holes that are adjacent to each other in the longitudinal direction do not intersect. Therefore, even when long screws are used, it is possible to prevent a disadvantageous situation in which the screws interfere with each other.

Furthermore, in the above-described first aspect, the bone plate may further include a plurality of screw holes that are disposed adjacent to each other at intervals in a direction intersecting the longitudinal direction or in a direction parallel to the longitudinal direction, at the end in the longitudinal direction.

By doing so, due to the force produced in the axis direction of the screw tightened into each of the screw holes, a high fixing force can be obtained with respect to the moment imposed in a direction intersecting the longitudinal direction of the bone plate. Providing the plurality of screw holes in the same plane ensures sufficient joining force of the bone plate and the bone.

Furthermore, in the above-described first aspect, the female thread may be a tapered female thread, or the female thread may be formed on an inner surface of the screw hole, which has a spherical shape.

By doing so, even when the axis of the screw is inclined with respect to the axis of the screw hole, the male thread and the female thread can be tightened over a wide range, thus obtaining a high fixing force.

Furthermore, according to a second aspect, the present invention provides a bone plate system including: any one of the above-described bone plates; and a plurality of screws or substantially cylindrical supporting bodies for fixing the bone plate to a bone, in which at least one of the screws or the substantially cylindrical supporting bodies has, on an outer surface of a head thereof, a male thread to be tightened into the female thread of the screw hole provided in the bone plate.

Furthermore, according to a third aspect, the present invention provides a bone plate system including: the above-described bone plate; and a plurality of screws or substantially cylindrical supporting bodies for fixing the bone plate to a bone, in which at least one of the screws or the substantially cylindrical supporting bodies has, on an outer surface of a head thereof, a tapered male thread to be tightened into the tapered female thread of the screw hole provided in the bone plate.

Furthermore, according to a fourth aspect, the present invention provides a bone plate system including: the above-described bone plate; and a plurality of screws or substantially cylindrical supporting bodies for fixing the bone plate to a bone, in which at least one of the screws or the substantially cylindrical supporting bodies has, on an outer surface of a spherical-shaped head thereof, a male thread to be tightened into the female thread on an inner surface of the spherical-shaped screw hole provided in the bone plate.

Furthermore, according to a fifth aspect, the present invention provides a bone plate system including: any one of the above-described bone plates; and a plurality of screws for fixing the bone plate to a bone, in which at least one of the screws is provided with a head that has a male thread to be tightened into the female thread of the screw hole provided in the bone plate and a shaft that has a male thread whose pitch is equal to that of the male thread provided on the head.

With the screw in which the pitch of the male thread on the head is equal to the pitch of the male thread on the shaft, the progress of the head tightened into the bone plate matches the progress of the shaft tightened into the bone. Therefore, the bone plate can be fixed to the bone without being distorted.

Furthermore, according to a sixth aspect, the present invention provides a bone plate system including: any one of the above-described bone plates; and a plurality of screws for fixing the bone plate to a bone, in which at least one of the screws is provided with a head that has a male thread to be tightened into the female thread of the screw hole provided in the bone plate and a shaft that has a male thread whose pitch is smaller than that of the male thread provided on the head.

With the screw in which the pitch of the male thread on the head is larger than the pitch of the male thread on the shaft, the progress of the head tightened into the bone plate is faster than the progress of the shaft tightened into the bone. Therefore, a load can be applied in directions in which the distance between the bone plate and the bone is increased.

Furthermore, according to a seventh aspect, the present invention provides a bone plate system including: any one of the above-described bone plates; and a plurality of screws for fixing the bone plate to a bone, in which at least one of the screws is provided with a head that has a male thread to be tightened into the female thread of the screw hole provided in the bone plate and a shaft that has a male thread whose pitch is larger than that of the male thread provided on the head.

With the screw in which the pitch of the male thread on the head is smaller than the pitch of the male thread on the shaft, the progress of the shaft tightened into the bone is faster than the progress of the head tightened into the bone plate. Therefore, a load can be applied in directions in which the distance between the bone plate and the bone is reduced.

In the above-described second to seventh aspects, the bone plate system may further include a different screw having a head whose external dimension is larger than an inner-diameter dimension of the screw hole and that is not provided with a male thread on an outer surface thereof.

When the plurality of screws that have the male threads on the heads thereof and the different screw that does not have the male thread on the head thereof are used together, it is possible to partially pull part of the bone toward the bone plate by using the different screw that does not have the male thread on the head thereof, while allowing the bone and the bone plate to be fixed relatively safely with high ease-of-handling by using the plurality of screws that have the male threads on the heads thereof.

Furthermore, in the above-described second to seventh aspects, the bone plate may be provided with at least one through-hole having a counterbore whose bearing surface is formed of a spherical inner surface or a cylindrical inner surface; and the bone plate system may further include a different screw having a head that is formed into a complementary shape to the bearing surface of the through-hole and that is not provided with a male thread on an outer surface thereof.

By inserting the different screw into the through-hole in the bone plate, even when the axis of the different screw is inclined with respect to the axis of the through-hole, the angle of the different screw inserted into the bone plate need not depend on the screw thread of the female thread. Furthermore, the degree of freedom of the angle of the different screw tightened into the bone can be improved.

### {Advantageous Effects of Invention}

According to the present invention, an object thereof is to provide a safe and high-ease-of-handle bone plate and bone plate system capable of tightening screws into a bone (in particular, a lower leg, a tibia) at desired angles while avoiding a troublesome bone-plate deforming task at an operation site and risks, such as the occurrence of metallic powder and implant fracture.

### {Brief Description of Drawings}

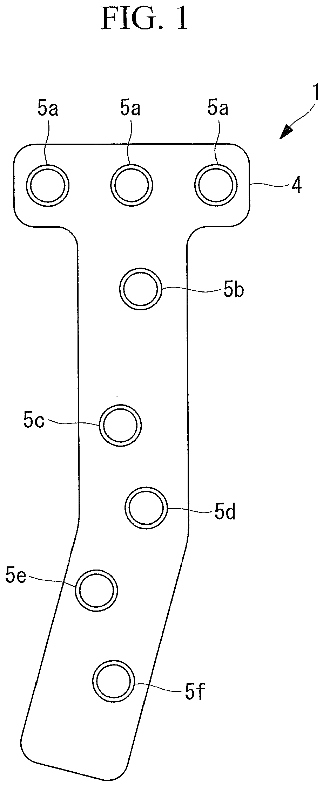

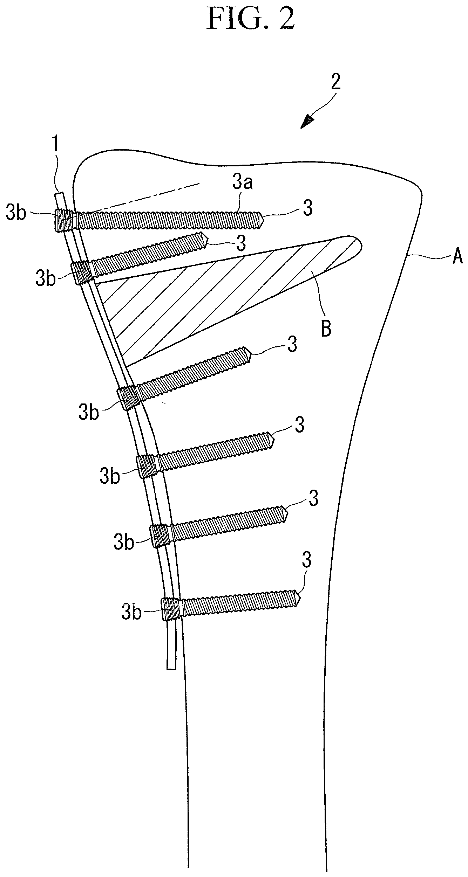

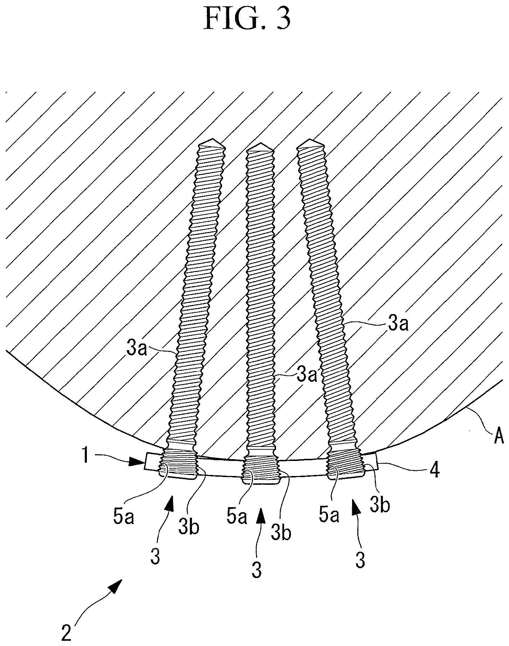

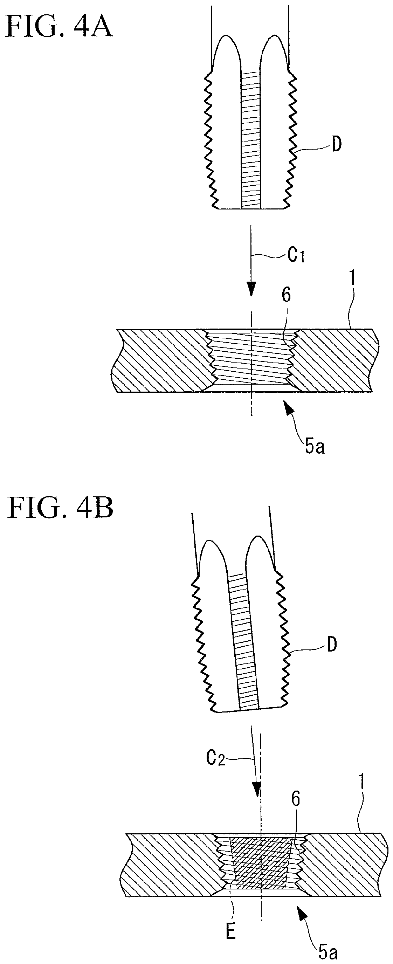

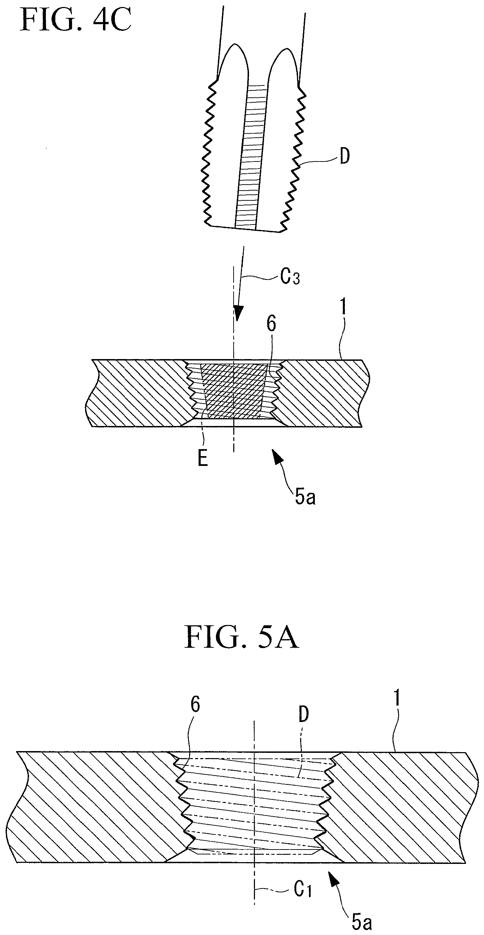

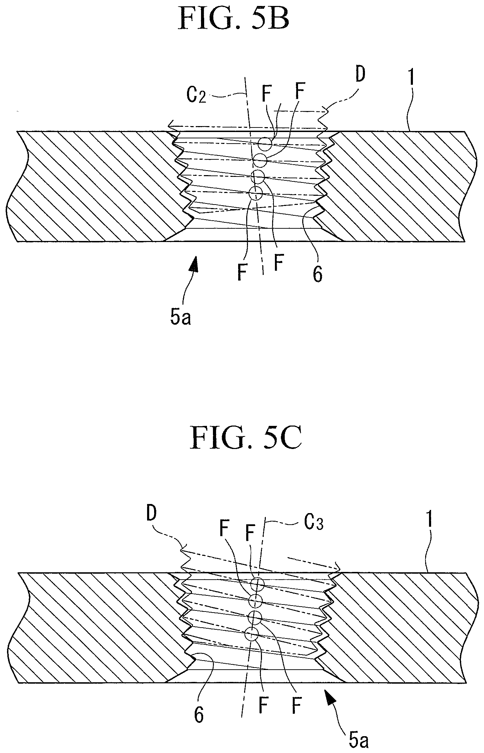

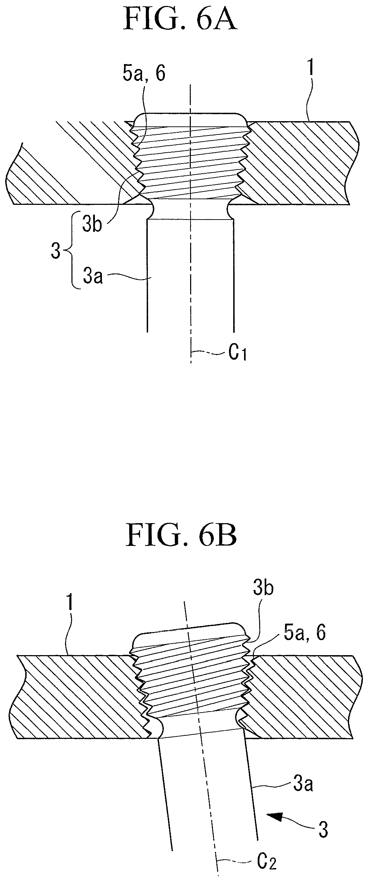

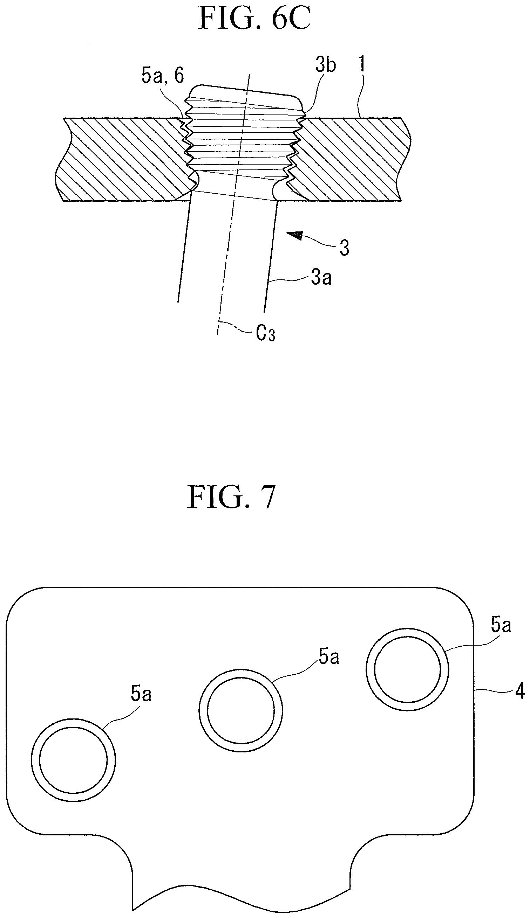

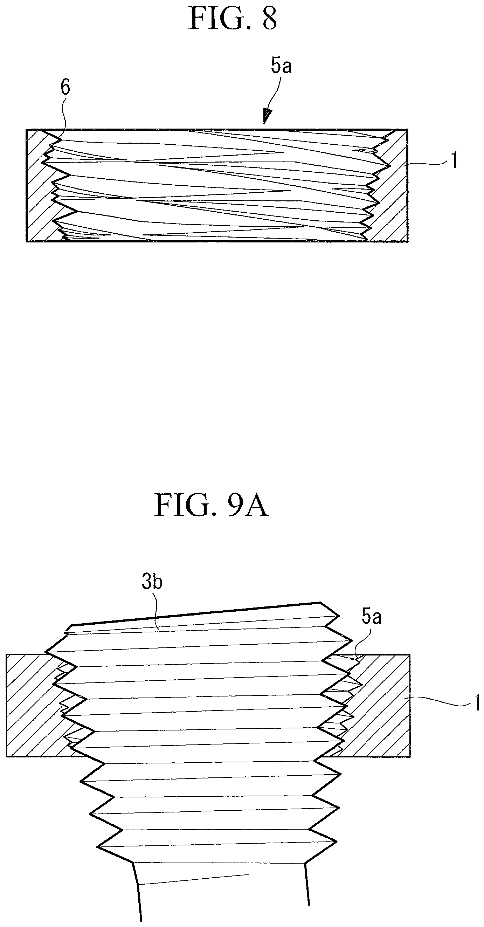

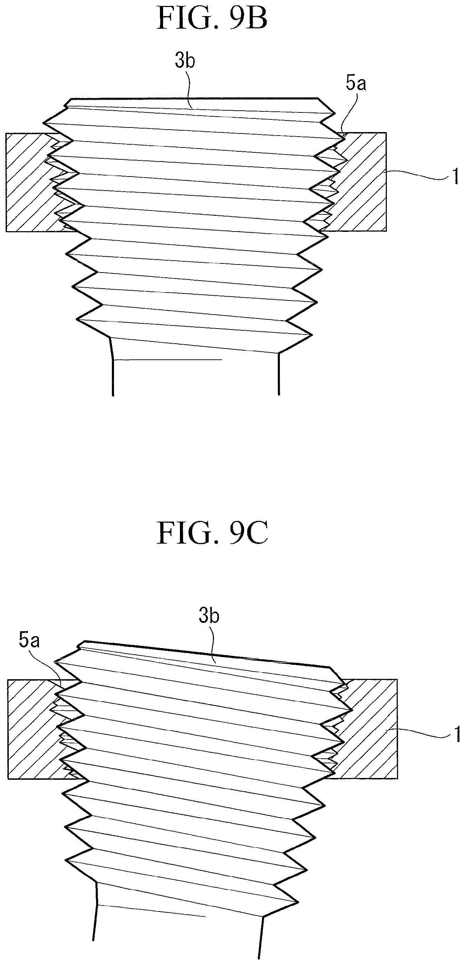

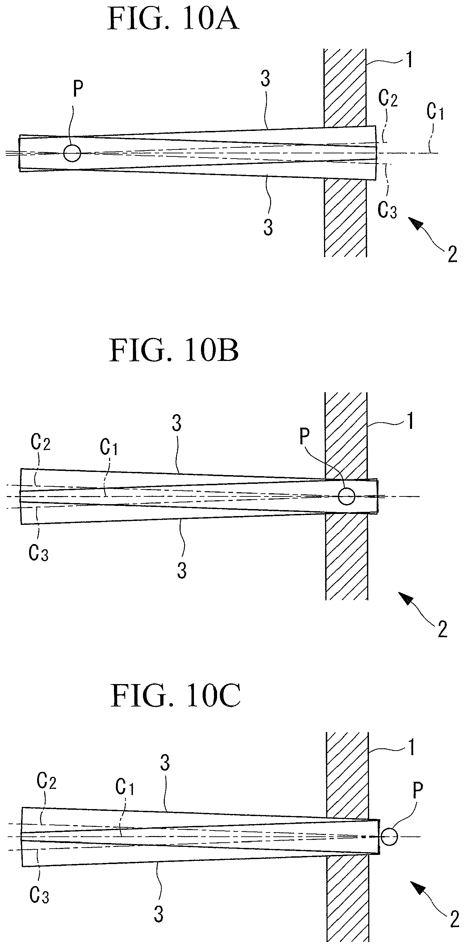

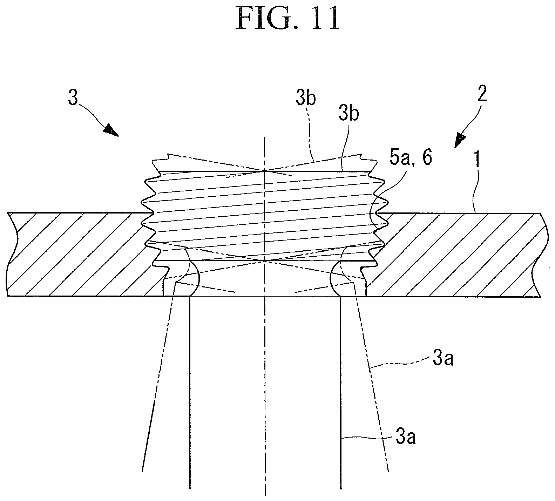

{Fig. 1} Fig. 1 is a front view showing a bone plate according to an embodiment of the present invention.
{Fig. 2} Fig. 2 is a longitudinal sectional view of a bone plate system according to this embodiment, the bone plate system having the bone plate shown in Fig. 1.
{Fig. 3} Fig. 3 is a longitudinal sectional view showing a wide portion of the bone plate in the bone plate system shown in Fig. 2.
{Fig. 4A} Fig. 4A is a partial longitudinal sectional view showing the direction of an axis C1 of a prepared hole, in tapping for a female thread to be provided in the wide portion of the bone plate shown in Fig. 1.
{Fig. 4B} Fig. 4B is a partial longitudinal sectional view showing the direction of an axis C2 inclined in one direction, in tapping of the female thread to be provided in the wide portion of the bone plate shown in Fig. 1.
{Fig. 4C} Fig. 4C is a partial longitudinal sectional view showing the direction of an axis C3 inclined in another direction, in tapping of the female thread to be provided in the wide portion of the bone plate shown in Fig. 1.
{Fig. 5A} Fig. 5A is a partial longitudinal sectional view showing tapping performed along the axis C1 of the prepared hole, in the tapping shown in Fig. 4A.
{Fig. 5B} Fig. 5B is a partial longitudinal sectional view showing a screw thread cut in the tapping direction of the axis C2, in the tapping shown in Fig. 4B.
{Fig. 5C} Fig. 5C is a partial longitudinal sectional view showing a screw thread cut in the tapping direction of the axis C3, in the tapping shown in Fig. 4C.
{Fig. 6A} Fig. 6A is a partial longitudinal sectional view showing a state in which a male thread portion of a screw in the bone plate system shown in Fig. 3 is tightened in the direction of the axis C1 of the screw hole.
{Fig. 6B} Fig. 6B is a partial longitudinal sectional view showing a state in which the male thread portion of the screw in the bone plate system shown in Fig. 3 is tightened in the direction of the axis C2 inclined with respect to the axis C1 of the screw hole.
{Fig. 6C} Fig. 6C is a partial longitudinal sectional view showing a state in which the male thread portion of the screw in the bone plate system shown in Fig. 3 is tightened in the direction of the axis C3 inclined with respect to the axis C1 of the screw hole.
{Fig. 7} Fig. 7 is a front view showing a modification of the bone plate shown in Fig. 1.
{Fig. 8} Fig. 8 is a longitudinal sectional view of a screw hole obtained after tapping is performed such that crests of a screw thread facing each other in the longitudinal direction of the bone plate are aligned with a 1/3-crest shift therebetween.
{Fig. 9A} Fig. 9A is a partial sectional view showing a state in which a screw is tightened into the screw hole shown in Fig. 8 in a certain axis direction.
{Fig. 9B} Fig. 9B is a partial sectional view showing a state in which a screw is tightened into the screw hole shown in Fig. 8 in another axis direction.
{Fig. 9C} Fig. 9C is a partial sectional view showing a state in which a screw is tightened into the screw hole shown in Fig. 8 in still another axis direction.
{Fig. 10A} Fig. 10A is a front view showing a state in which an intersection point of the axes for tapping is located closer to a bone than to the bone plate, according to a modification of the bone plate shown in Fig. 1.
{Fig. 10B} Fig. 10B is a front view showing a state in which an intersection point of the axes for tapping is located at a position in the plate thickness of the bone plate, according to the modification of the bone plate shown in Fig. 1.
{Fig. 10C} Fig. 10C is a front view showing a state in which an intersection point of the axes for tapping is located on the opposite of the bone plate from the bone, according to the modification of the bone plate shown in Fig. 1.
{Fig. 11} Fig. 11 is a partial longitudinal sectional view showing a modification of the bone plate shown in Fig. 1 and the bone plate system shown in Fig. 2.

### {Description of Embodiments}

A bone plate 1 and a bone plate system 2 according to an embodiment of the present invention will be described below with reference to the drawings.

As shown in Fig. 2, the bone plate system 2 of this embodiment includes the bone plate 1 and a plurality of screws 3 for fixing the bone plate 1 to a side surface of a bone, for example, a tibia A.

The bone plate 1 of this embodiment is an elongated band-plate member to be fixed to the side surface of a tibia that has been cut in a high tibial osteotomy for knee osteoarthritis. The bone plate 1 has a slightly curved shape to conform to a typical surface shape of a tibia, so as to be able to follow the curved shape of the side surface of the tibia at a position extending from the shaft of the tibia toward an end thereof.

The bone plate 1 includes, at one end thereof in the longitudinal direction, a wide portion 4 whose width dimension is increased in a direction perpendicular to the longitudinal direction. The bone plate 1 is formed into a substantially T-shape as a whole.

The bone plate 1 is provided with a plurality of, for example, six, screw holes 5a, 5b, 5c, 5d, 5e, and 5f arranged at intervals in the longitudinal direction. The screw holes 5a, 5b, 5c, 5d, 5e, and 5f are arranged in this order in the longitudinal direction from the wide portion 4 side. Furthermore, in the wide portion 4, a plurality of, for example, three, screw holes 5a are arranged at intervals in the direction perpendicular to the longitudinal direction.

Among the six screw holes 5a, 5b, 5c, 5d, 5e, and 5f arranged at intervals in the longitudinal direction, the five screw holes 5b, 5c, 5d, 5e, and 5f, but not the screw hole 5a provided in the wide portion 4, are formed of usual tapered threads. These screw holes 5b, 5c, 5d, 5e, and 5f each have an inner diameter that gradually decreases in the plate thickness direction from one side toward the other side, specifically, toward a surface that is made to face the tibia A. The screw holes 5a, 5b, 5c, 5d, 5e, and 5f each have an axis in the plate thickness direction of the bone plate 1.

As shown in Fig. 1, the positions of the screw holes 5a, 5b, 5c, 5d, 5e, and 5f, which are adjacent in the longitudinal direction, are slightly shifted in the direction perpendicular to the longitudinal direction, so that their axes are located at almost parallel or shifted positions. Thus, even if the lengths of the screws 3 to be tightened into the screw holes 5a, 5b, 5c, 5d, 5e, and 5f are sufficiently long, the screws 3 do not interfere with each other.

As shown in Figs. 4A, 4B, and 4C, in the bone plate 1 of this embodiment, the three screw holes 5a provided in the wide portion 4 each have a female thread 6 that is formed when a prepared hole is subjected to tapping using a tap D in the direction of an axis C1 of the prepared hole and in the directions of two axes C2 and C3 inclined with respect to the axis C1. Thus, the screw thread of the female thread 6 formed first is partially cut across to form the new female thread 6 in the screw hole 5a. Specifically, as shown in Figs. 4B and 4C, a portion of the screw thread located in a region E mainly indicated by the hatching is partially cut by the tapping performed in multiple directions. Depending on the desired angle of the screw 3, a portion of the screw thread located outside the region E may also be cut as long as the fixing strength is not reduced.

Specifically, after tapping is performed using the tap D along the axis C1, as shown in Fig. 5A, tapping is performed along the inclined axes C2 and C3, as shown in Figs. 5B and 5C. Thus, the screw thread of the female thread 6 formed in the screw hole 5a is cut at portions indicated by reference numeral F to form the female thread 6.

In this embodiment, the axes C2 and C3 for tapping are inclined within a plane that includes the axis C1 of the prepared hole for the screw hole 5a and that extends in the longitudinal direction of the bone plate 1. The angles of inclination of the axes C2 and C3 for tapping vary according to the pitch of the female thread 6 formed in the screw hole 5a. In this embodiment, the tap is inserted while being inclined such that crests of the screw thread facing each other in the longitudinal direction of the bone plate 1 are aligned with a 1-crest shift therebetween. Thus, the screw 3 to be tightened into the screw hole 5a can be tightened while being inclined in the longitudinal direction of the bone plate 1, as shown in Fig. 2 and Figs. 6.

As shown in Figs. 2 and 3, the screws 3 are formed in an elongated round bar shape. Bone-fixing male threads 3a to be tightened into prepared holes (not shown) formed in the tibia A are provided on outer peripheries of shafts of the screws 3. Plate-fixing male threads 3b formed of tapered threads to be tightened into the screw holes 5a, 5b, 5c, 5d, 5e, and 5f of the bone plate 1 are provided on outer peripheries of heads of the screws 3.

Advantageous effects of the thus-structured bone plate 1 and bone plate system 2 according to this embodiment will be described below.

To perform a high tibial osteotomy for knee osteoarthritis by using the bone plate system 2 of this embodiment, a cut is made in the tibia A from the medial surface toward an outer side in a direction inclined with respect to the long axis of the tibia A, and the cut surface is dilated by using a predetermined instrument. Then, in a state in which a wedge-shaped filling material B is inserted into the dilated cut surface, the bone plate 1 is placed on the medial surface of the tibia A such that the one screw hole 5b, which is the closest to the wide portion 4, and the four screw holes 5c, 5d, 5e, and 5f are arranged with the cut interposed therebetween.

Next, prepared holes are formed in the screw holes 5b, 5c, 5d, 5e, and 5f. At this time, for the screw holes 5b, 5c, 5d, 5e, and 5f, which are not provided in the wide portion 4, prepared holes are formed in the directions of the axes of the screw holes 5b, 5c, 5d, 5e, and 5f. On the other hand, for the three screw holes 5a, which are provided in the wide portion 4, prepared holes are formed so as to be almost parallel to an end surface of the tibia A. Then, the bone-fixing male threads 3a of the screws 3 are made to pass through the screw holes 5a, 5b, 5c, 5d, 5e, and 5f and are tightened into the prepared holes, and, finally, the plate-fixing male threads 3b, which are provided on the heads of the screws 3, are tightened into the screw holes 5a, 5b, 5c, 5d, 5e, and 5f.

Thus, the bone plate 1 is fixed to the tibia A while being disposed along the surface of the tibia A. In this case, since the female threads of the screw holes 5a, 5b, 5c, 5d, 5e, and 5f and the plate-fixing male threads 3b of the screws 3 are formed of tapered threads, the fixing force is increased as tightening progresses, thus making it possible for the bone plate 1 to be more securely fixed to the surface of the tibia A.

Furthermore, according to the bone plate 1 and the bone plate system 2 of this embodiment, the angles of the male threads 3b of the screws 3 tightened into the female threads of the screw holes 5a, which are provided in the wide portion 4, can be selected according to the following three tightening methods. In a first method, as shown in Fig. 6A, the screws 3 are tightened into the screw holes 5a at angles at which the axes of the screws 3 are aligned with the axes of the screw holes 5a. In second and third methods, as shown in Fig. 6B and Fig. 6C, the screws 3 are tightened into the screw holes 5a at angles obtained by shifting the tightening position where crests of the female thread 6 face each other in the longitudinal direction of the bone plate 1 by 1 crest in either direction.

In high tibial osteotomy for knee osteoarthritis, the extent of dilation of a cut made in the tibia A varies according to the degree of deformation of the tibia A, and the surface shape of the tibia A differs from patient to patient. According to the bone plate 1 and the bone plate system 2 of this embodiment, an advantage is afforded in that, even when the same-shaped bone plate 1 is used for all patients, the screws 3 can be set in appropriate fixing directions by suitably selecting from among the methods for tightening the screws 3 into the screw holes 5a.

Specifically, when the same-shaped bone plate 1 is used for all patients, if the fixing directions of the screws 3 are the same, the screws 3 for fixing the wide portion 4 might be disposed in directions in which they pass through the end surface of the tibia A, or the screws 3 might be disposed in directions in which they pass through the filling material B. On the other hand, according to this embodiment, such a disadvantageous situation does not occur; it is possible to tighten the screws 3 for fixing the wide portion 4, in directions almost parallel to the end surface of the tibia A, without forcing a practitioner to perform the troublesome task of deforming the bone plate 1 at an operation site.

Furthermore, according to the bone plate 1 and the bone plate system 2 of this embodiment, even if the directions in which the screws 3 are tightened into the screw holes 5a, which are provided in the wide portion 4, are inclined in the longitudinal direction, because tapping is performed in advance in a plurality of different directions, it is possible to prevent the female threads 6 from being stripped through tightening, thus preventing the occurrence of metallic powder.

Furthermore, since the female thread portions 6 of the screw holes 5a of the bone plate 1 and the plate-fixing male threads 3b of the screws 3 are formed of tapered threads, it is possible to improve the fixing force between the screws 3 and the bone plate 1, while ensuring the degree of freedom for tightening the male threads 3b into the female thread portions 6. Therefore, there is an advantage in that, even if the screws 3 are inclined with respect to the axes of the screw holes 5a, the screws 3 and the bone plate 1 can be tightened more securely.

Furthermore, in this embodiment, since the wide portion 4 is provided in the bone plate 1, and the three screw holes 5a are provided in the wide portion 4 at intervals in the direction perpendicular to the longitudinal direction of the bone plate 1, a moment imposed on the bone plate 1 can be received by the tension of the three screws 3 tightened into the screw holes 5a. Therefore, even if a twist is applied to the tibia A, the bone plate 1 can be maintained in the securely-fixed state.

In this embodiment, although the bone plate 1 is formed into a substantially T-shape, the shape thereof is not limited thereto. For example, the bone plate 1 may be formed into a substantially L-shape, a substantially J-shape, a substantially I-shape, a substantially S-shape, a substantially Y-shape, a substantially γ-shape, or a substantially r-shape (T-shape in the drawing). Furthermore, although the three screw holes 5a are provided in the wide portion 4, the screw holes 5a may be provided in the bone plate 1 such that two or more (desirably three or more) screws 3 are fixed to one piece of the bone. With this structure, almost the same effect as that of this embodiment can be afforded. Furthermore, in the example shown in Fig. 1, the bone plate 1 has a curved shape in order to prevent an end portion of the bone plate 1 on the side opposite to the wide portion 4 from protruding outward from the tibia when the bone plate 1 is fixed to the tibia A; however, the shape thereof is not limited thereto.

Furthermore, although the three screw holes 5a in the wide portion 4 are arranged at intervals in the direction perpendicular to the long axis, instead of this, the three screw holes 5a may be arranged at intervals in a direction that intersects the longitudinal direction at a 90 degree angle or less, as shown in Fig. 7. By doing so, also in the wide portion 4, the axes of the screw holes 5a can be located at mutually shifted positions. Thus, because the axes do not intersect, even when long screws 3 are used, they can be tightened so as not to interfere with each other.

Furthermore, in this embodiment, although tapping is performed in the directions of the axes C2 and C3 such that crests of the screw thread facing each other in the longitudinal direction of the bone plate 1 are aligned with a 1-crest shift therebetween, instead of this, for example, tapping may be performed in the directions of the axes C2 and C3 such that crests of the screw thread facing each other in the longitudinal direction of the bone plate 1 are aligned with a 0.5-crest shift therebetween. Specifically, tapping may be performed so as to form a screw thread having imperfect crests, such as 0.5 crests and 1.5 crests. By doing so, the fixing angle of the screw 3 can be changed by a minute angle.

In this case, although a portion of the screw thread located outside the region E shown in Figs. 4B and 4C is also cut, the tapping may be performed as long as a required fixing strength according to the place where the bone plate 1 is used can be realized. For example, as shown in Fig. 8, tapping may be performed in the directions of the axes C1, C2, and C3 to form the screw hole 5a such that crests of the screw thread facing each other in the longitudinal direction of the bone plate 1 are aligned with a 1/3-crest shift therebetween. According to the screw hole 5a shown in Fig. 8, the fixing angle of the screw 3 can be changed by a minute angle, as shown in Figs. 9A, 9B, and 9C.

Furthermore, in this embodiment, although a description has been given of a case where tightening is performed in a state in which the facing crests of the screw thread of the female thread portion 6 are shifted by 1 crest, instead of this tightening may be performed in a state in which they are shifted by 2 crests after the pitch of the screw thread is sufficiently reduced.

Furthermore, in this embodiment, the intersection point P of the axes C1, C2, and C3 for tapping performed for an identical prepared hole can be desirably set, as shown in Figs. 10A, 10B, and 10C. Specifically, as shown in Fig. 10A, the axes C1, C2, and C3 are made to intersect (the intersection point P) at a distal side of the screw 3, thereby making it possible to tighten the screw 3 so as to always make it pass through the identical point P at the distal side and to finely adjust the locking position of the bone plate 1. Furthermore, as shown in Fig. 10B, the intersection point P of the axes C1, C2, and C3 is located at an intermediate position in the plate thickness direction of the bone plate 1, thereby making it possible to adjust the angle of the screw 3 without moving the locking position of the bone plate 1. Furthermore, as shown in Fig. 10C, the intersection point P of the axes C1, C2, and C3 is located on the opposite of the bone plate 1 from the bone, thereby making it possible to change the angle of the screw 3 and to finely adjust the locking position, simultaneously.

Furthermore, in this embodiment, although the female thread portions 6 of the screw holes 5a, which are provided in the wide portion 4, and the plate-fixing male threads 3b of the screws 3 to be tightened thereinto are formed of tapered threads, instead of this, as shown in Fig. 11, the female thread portions 6 and the male threads 3b that each have, on a substantially-spherically-curved inner surface and outer surface, a screw thread extending toward substantially the center of the sphere may be adopted. By doing so, an advantage is afforded in that, even when the axis of the screw 3 is inclined with respect to the axis of the screw hole 5a, it is possible to ensure a large contact range of the female thread portion 6 and the plate-fixing male thread 3b and to obtain a sufficient fixing force. Furthermore, irrespective of the shape of the head of the screw 3, the pitch of the male thread 3b, provided on the head of the screw 3, and the pitch of the male thread 3a, provided on the shaft thereof, may be set equal, for example. By doing so, the ease-of-fixing the screw 3 can be improved.

Furthermore, in this embodiment, although the axes C2 and C3 for tapping are inclined in a plane in the longitudinal direction, thereby allowing the screw 3 to be inclined in this plane, the screw hole 5a can be structured such that the axis for tapping is inclined in a different direction, thereby allowing the screw 3 to be inclined in another direction. By doing so, the tightening angle of the screw can be selected from not only two directions but also three or more directions. For example, the tightening angle of the screw may be selected from two tightening methods or four or more tightening methods, including an angle obtained by shifting the tightening position where crests of the female thread 6 face each other in a width direction or a diagonal direction of the bone plate 1 by 1 crest in either direction.

Furthermore, in high tibial osteotomy, it is necessary to apply compression (pressure) to the bone plate 1 to improve the stability of the entire bone plate 1 and to apply an appropriate mechanical load to a portion of the bone that has been cut, in some cases. Specifically, the stability of the bone plate 1 can be improved by providing a mechanism that enables adjustment of the distance between the bone and the bone plate 1.

As one method for adjusting the distance between the bone and the bone plate 1, for example, the screws 3 may be used together with different screws (not shown), in which the external dimension of the head of each of the different screws is larger than the inner-diameter dimension of the screw hole 5b etc., and in which a male thread is not provided on the head thereof. Specifically, in the screw hole 5b etc., the different screws, which have male threads provided only on the shafts thereof to be tightened into the bone, may be used instead of the screws 3.

The screws 3 stop pulling the bone toward the bone plate 1 when the male threads 3a, provided on the heads, are completely tightened into the screw holes 5a in the bone plate 1. On the other hand, in general, the different screws, which do not have male threads on the heads thereof, can mechanically stabilize the fixed portion. Furthermore, as the tightening torque is increased, those different screws pull the bone toward the bone plate, thereby improving the effect of mechanically stabilizing the fixed portion.

Therefore, when the plurality of screws 3, which have the male threads 3a on the heads thereof, and the different screws, which do not have male threads on the heads thereof, are used together, it is possible to strongly pull part of the bone toward the bone plate 1 by using the different screws, while allowing the bone and the bone plate 1 to be fixed relatively safely with high ease-of-handling by using the plurality of screws 3. For example, in usual cases, the screw 3 may be tightened into the screw hole 5b, which is located close to the wide portion 4 in the longitudinal direction of the bone plate 1, and a different screw, which does not have the male thread on the head thereof, may be used, instead of the screw 3, depending on the case, for example, when it is necessary to strongly pull the bone toward the bone plate 1 because a cut portion of the bone is unstable. The head of the different screw may be formed into a substantially cylindrical shape, a substantially conic shape, or a substantially spherical shape, for example.

Furthermore, at least one through-hole (not shown) with a counterbore may be provided in the bone plate 1, and the different screw (not shown), which does not have the male thread on the outer surface of the head, may be inserted into the through-hole and tightened into the bone. In this case, it is desirable that a bearing surface of the through-hole have a spherical inner surface or a cylindrical inner surface, and that the head of the different screw have a complementary shape to the bearing surface of the through-hole. By doing so, even when the axis of the different screw is inclined with respect to the axis of the through-hole, the angle of the different screw inserted into the bone plate 1 need not depend on the screw thread of the female thread. Furthermore, by forming the bearing surface of the through-hole and the head of the different screw into complementary shapes, the degree-of-freedom of the angle of the different screw tightened into the bone can be improved.

As another method for adjusting the distance between the bone and the bone plate 1, a difference may be intentionally provided between the pitch of the male thread 3b, provided on the head of the screw 3, and the pitch of the male thread 3a, provided on the shaft thereof. For example, the pitch of the male thread 3b on the head may be set larger than the pitch of the male thread 3a on the shaft. By doing so, because progress of the head to be tightened into the bone plate 1 is faster than progress of the shaft to be tightened into the bone, a load can be applied in directions in which the distance between the bone and the bone plate 1 is increased. Furthermore, the pitch of the male thread 3b on the head may be set smaller than the pitch of the male thread 3a on the shaft. By doing so, because progress of the shaft to be tightened into the bone is faster than progress of the head to be tightened into the bone plate 1, a load can be applied in directions in which the distance between the bone and the bone plate 1 is reduced. In this way, when the pitch of the male thread 3b on the head and the pitch of the male thread 3a on the shaft are set different, it is possible to fix the angle between the screw 3 and the bone plate 1 and also to apply compression to the bone plate 1.

Furthermore, in this embodiment, although a description has been given of the screws 3 as an example, instead of this, for example, long substantially cylindrical bar-like members (substantially cylindrical supporting bodies) that have the male threads 3a on the outer peripheries of the heads and do not have male threads on the outer peripheries of the shafts may be adopted.

Furthermore, although a description has been given of tapping as a method for forming the female thread 6 in the screw hole 5a, the female thread 6 may be formed in a prepared hole by cutting a screw thread in the direction of the axis C1 of the prepared hole and along one or more axes inclined with respect to the axis C1. For example, the female thread 6 may be formed in the screw hole 5a through turning, milling, or electric discharge machining.

### {Reference Signs List}

- A: tibia (bone)
- 1: bone plate
- 2: bone plate system
- 3: screw
- 3b: male thread (male thread portion)
- 5a, 5b, 5c, 5d, 5e, 5f: screw hole
- 6: female thread

## Claims

1. A bone plate (1) that is configured to be formed into a band plate disposed along a bone (A) surface, the bone plate (1) comprising a plurality of screw holes (5a-5f) penetrating in a plate thickness direction,
wherein at least one of the screw holes (5a) that is located at an end in a longitudinal direction has a female thread (6),
wherein the female thread (6) is formed by cutting a screw thread along an axis (C₁) of a prepared hole and two or more axes (C₂, C₃) inclined with respect to the axis (C₁) of the prepared hole, each of the axes (C₁, C₂, C₃) having a respective direction, and wherein cutting is performed by tapping in the directions of the two or more inclined axes (C₂, C₃) such that crests of the screw thread facing each other in the longitudinal direction are aligned with a crest shift therebetween, wherein the crest shift is selected from the group of 1/3, 0.5, 1 and 1.5 crests.

2. The bone plate (1) according to claim 1, wherein axes (C₂, C₃) of the plurality of screw holes (5a-5f) disposed adjacent to each other in the longitudinal direction are located at mutually shifted positions or at parallel positions at intervals in a direction perpendicular to the longitudinal direction.

3. The bone plate (1) according to claim 1, further comprising a plurality of screw holes (5a-5f) that are disposed adjacent to each other at intervals in a direction intersecting the longitudinal direction or in a direction parallel to the longitudinal direction, at the end in the longitudinal direction.

4. The bone plate (1) according to claim 1, wherein the female thread (6) is a tapered female thread.

5. The bone plate (1) according to claim 1, wherein the female thread (6) is formed on an inner surface of the screw hole (5a), which has a spherical shape.

6. A bone plate system (2) comprising:
a bone plate (1) according to any one of claims 1 to 5; and
a plurality of screws (3) or substantially cylindrical supporting bodies for fixing the bone plate (1) to a bone (A),
wherein at least one of the screws (3) or the substantially cylindrical supporting bodies has, on an outer surface of a head thereof, a male thread (3b) configured to be tightened into the female thread (6) of a corresponding screw hole (5a-5f) provided in the bone plate (1).

7. A bone plate system (2) comprising:
a bone plate (1) according to claim 4; and
a plurality of screws (3) or substantially cylindrical supporting bodies for fixing the bone plate (1) to a bone (A),
wherein at least one of the screws (3) or the substantially cylindrical supporting bodies has, on an outer surface of a head thereof, a tapered male thread (3b) configured to be tightened into the tapered female thread (6) of a corresponding screw hole (5a-5f) provided in the bone plate (1).

8. A bone plate system (2) comprising:
a bone plate (1) according to claim 5; and
a plurality of screws (3) or substantially cylindrical supporting bodies for fixing the bone plate (1) to a bone (A),
wherein at least one of the screws (3) or the substantially cylindrical supporting bodies has, on an outer surface of a spherical-shaped head thereof, a male thread (3b) configured to be tightened into the female thread (6) on an inner surface of a corresponding spherical-shaped screw hole (5a-5f) provided in the bone plate (1).

9. A bone plate system (2) comprising:
a bone plate (1) according to any one of claims 1 to 5; and
a plurality of screws (3) for fixing the bone plate (1) to a bone (A),
wherein at least one of the screws (3) is provided with a head that has a male thread (3b) configured to be tightened into the female thread (6) of a corresponding screw hole (5a-5f) provided in the bone plate (1) and a shaft that has a male thread (3a) whose pitch is equal to that of the male thread provided on the head.

10. A bone plate system (2) comprising:
a bone plate (1) according to any one of claims 1 to 5; and
a plurality of screws (3) for fixing the bone plate (1) to a bone (A),
wherein at least one of the screws (3) is provided with a head that has a male thread (3a) configured to be tightened into the female thread (6) of a corresponding screw hole (5a-5f) provided in the bone plate (1) and a shaft that has a male thread (3a) whose pitch is smaller than that of the male thread (3b) provided on the head.

11. A bone plate system (2) comprising:
a bone plate (1) according to any one of claims 1 to 5; and
a plurality of screws (3) for fixing the bone plate (1) to a bone (A),
wherein at least one of the screws (3) is provided with a head that has a male thread (3b) configured to be tightened into the female thread (6) of a corresponding screw hole (5a-5f) provided in the bone plate (1) and a shaft that has a male thread (3a) whose pitch is larger than that of the male thread (3b) provided on the head.

12. A bone plate system (2) according to any one of claims 6 to 11, further comprising a different screw having a head whose external dimension is larger than an inner-diameter dimension of a corresponding screw hole and that is not provided with a male thread on an outer surface thereof.

13. A bone plate system (2) according to any one of claims 6 to 11,
wherein the bone plate (1) is provided with at least one through-hole having a counterbore whose bearing surface is formed of a spherical inner surface or a cylindrical inner surface; and
the bone plate system (2) further comprises a different screw having a head that is formed into a complementary shape to the bearing surface of the through-hole and that is not provided with a male thread on an outer surface thereof.

## Patentansprüche

1. Knochenplatte (1), die konfiguriert ist, zu einer entlang einer Oberfläche eines Knochens (A) angeordneten Bandplatte geformt zu werden, wobei die Knochenplatte (1) eine Mehrzahl von Schraubenlöchern (5a-5f) umfasst, die in Richtung einer Plattendicke verlaufen,
wobei mindestens eines der Schraubenlöcher (5a), das sich an einem Ende in einer Längsrichtung befindet, ein Innengewinde (6) aufweist,
wobei das Innengewinde (6) durch Schneiden eines Schraubengewindes entlang einer Achse (C₁) eines vorbereiteten Lochs und zwei oder mehr Achsen (C₂, C₃), die in Bezug auf die Achse (C₁) des vorbereiteten Lochs geneigt sind, gebildet ist, wobei jede der Achsen (C₁, C₂, C₃) eine jeweilige Richtung aufweist, und wobei das Schneiden durch Gewindeschneiden in den Richtungen der zwei oder mehr geneigten Achsen (C₂, C₃) durchgeführt wird, so dass Scheitelpunkte des Schraubengewindes, die einander in der Längsrichtung zugewandt sind, mit einer Scheitelpunktverschiebung dazwischen ausgerichtet sind, wobei die Scheitelpunktverschiebung ausgewählt ist aus der Gruppe von 1/3, 0,5, 1 und 1,5 Scheitelpunkten.

2. Knochenplatte (1) nach Anspruch 1, wobei die Achsen (C₂, C₃) der Mehrzahl von Schraubenlöchern (5a-5f), die in der Längsrichtung nebeneinander angeordnet sind, an zueinander versetzten Positionen oder an parallelen Positionen in Abständen in einer Richtung senkrecht zur Längsrichtung angeordnet sind.

3. Knochenplatte (1) nach Anspruch 1, die ferner eine Mehrzahl von Schraubenlöchern (5a-5f) umfasst, die an dem Ende in der Längsrichtung in einer die Längsrichtung schneidenden Richtung oder in einer zur Längsrichtung parallelen Richtung in Abständen nebeneinander angeordnet sind.

4. Knochenplatte (1) nach Anspruch 1, wobei das Innengewinde (6) ein konisches Innengewinde ist.

5. Knochenplatte (1) nach Anspruch 1, wobei das Innengewinde (6) an einer Innenfläche des Schraubenlochs (5a) ausgebildet ist, das eine kugelförmige Form aufweist.

6. Knochenplattensystem (2), das umfasst:
eine Knochenplatte (1) nach einem der Ansprüche 1 bis 5; und
eine Mehrzahl von Schrauben (3) oder im Wesentlichen zylindrischen Stützkörpern zur Befestigung der Knochenplatte (1) an einem Knochen (A),
wobei mindestens eine der Schrauben (3) oder der im Wesentlichen zylindrischen Stützkörper an einer Außenfläche ihres/seines Kopfes ein Außengewinde (3b) aufweist, das konfiguriert ist, in das Innengewinde (6) eines entsprechenden Schraubenlochs (5a-5f), das in der Knochenplatte (1) vorgesehen ist, eingeschraubt zu werden.

7. Knochenplattensystem (2), das umfasst:
eine Knochenplatte (1) nach Anspruch 4; und
eine Mehrzahl von Schrauben (3) oder im Wesentlichen zylindrischen Stützkörpern zur Befestigung der Knochenplatte (1) an einem Knochen (A),
wobei mindestens eine der Schrauben (3) oder der im Wesentlichen zylindrischen Stützkörper an einer Außenfläche ihres/seines Kopfes ein konisches Außengewinde (3b) aufweist, das konfiguriert ist, in das konische Innengewinde (6) eines entsprechenden Schraubenlochs (5a-5f), das in der Knochenplatte (1) vorgesehen ist, eingeschraubt zu werden.

8. Knochenplattensystem (2), das umfasst:
eine Knochenplatte (1) nach Anspruch 5; und
eine Mehrzahl von Schrauben (3) oder im Wesentlichen zylindrischen Stützkörpern zur Befestigung der Knochenplatte (1) an einem Knochen (A),
wobei mindestens eine der Schrauben (3) oder der im Wesentlichen zylindrischen Stützkörper an einer Außenfläche ihres/seines kugelförmigen Kopfes ein Außengewinde (3b) aufweist, das konfiguriert ist, in das Innengewinde (6) auf einer Innenfläche eines entsprechenden kugelförmigen Schraubenlochs (5a-5f), das in der Knochenplatte (1) vorgesehen ist, eingeschraubt zu werden.

9. Knochenplattensystem (2), das umfasst:
eine Knochenplatte (1) nach einem der Ansprüche 1 bis 5; und
eine Mehrzahl von Schrauben (3) zur Befestigung der Knochenplatte (1) an einem Knochen (A),
wobei mindestens eine der Schrauben (3) mit einem Kopf, der ein Außengewinde (3b) aufweist, das konfiguriert ist, in das Innengewinde (6) eines entsprechenden Schraubenlochs (5a-5f), das in der Knochenplatte (1) vorgesehen ist, eingeschraubt zu werden, und einem Schaft versehen ist, der ein Außengewinde (3a) aufweist, dessen Steigung gleich derjenigen des Außengewindes ist, das an dem Kopf vorgesehen ist.

10. Knochenplattensystem (2), das umfasst:
eine Knochenplatte (1) nach einem der Ansprüche 1 bis 5; und
eine Mehrzahl von Schrauben (3) zur Befestigung der Knochenplatte (1) an einem Knochen (A),
wobei mindestens eine der Schrauben (3) mit einem Kopf, der ein Außengewinde (3a) aufweist, das konfiguriert ist, in das Innengewinde (6) eines entsprechenden Schraubenlochs (5a-5f), das in der Knochenplatte (1) vorgesehen ist, eingeschraubt zu werden, und einem Schaft versehen ist, der ein Außengewinde (3a) aufweist, dessen Steigung kleiner ist als die des Außengewindes (3b), das an dem Kopf vorgesehen ist.

11. Knochenplattensystem (2), das umfasst:
eine Knochenplatte (1) nach einem der Ansprüche 1 bis 5; und
eine Mehrzahl von Schrauben (3) zur Befestigung der Knochenplatte (1) an einem Knochen (A),
wobei mindestens eine der Schrauben (3) mit einem Kopf, der ein Außengewinde (3b) aufweist, das konfiguriert ist, in das Innengewinde (6) eines entsprechenden Schraubenlochs (5a-5f), das in der Knochenplatte (1) vorgesehen ist, eingeschraubt zu werden, und einem Schaft versehen ist, der ein Außengewinde (3a) aufweist, dessen Steigung größer ist als die des Außengewindes (3b), das an dem Kopf vorgesehen ist.

12. Knochenplattensystem (2) nach einem der Ansprüche 6 bis 11, das ferner eine andere Schraube mit einem Kopf umfasst, dessen Außenabmessung größer ist als eine Innendurchmesserabmessung eines entsprechenden Schraubenlochs und der an seiner Außenfläche nicht mit einem Außengewinde versehen ist.

13. Ein Knochenplattensystem (2) nach einem der Ansprüche 6 bis 11,
wobei die Knochenplatte (1) mit mindestens einem Durchgangsloch mit einer Senkbohrung versehen ist, deren Auflagefläche aus einer kugelförmigen Innenfläche oder einer zylindrischen Innenfläche gebildet ist; und
das Knochenplattensystem (2) ferner eine andere Schraube umfasst, die einen Kopf aufweist, der komplementär zu der Auflagefläche des Durchgangslochs geformt ist und der an seiner Außenfläche kein Außengewinde aufweist.

## Revendications

1. Plaque vissée (1) qui est conçue pour pouvoir revêtir la forme d'une plaque en bande disposée le long d'une face osseuse (A), la plaque vissée (1) comprenant une pluralité de trous de vis (5a-5f) pénétrant dans un sens de l'épaisseur de la plaque,
dans lequel au moins l'un des trous de vis (5a) qui est situé à une extrémité dans un sens de la longueur a un filetage intérieur (6),
dans lequel le filetage intérieur (6) est formé par découpage d'un filet de vis le long d'un axe (C₁) pour un trou préparé et au moins deux axes (C₂, C₃) inclinés par rapport à l'axe (C₁) du trou préparé, chacun des axes (C₁, C₂, C₃) ayant un sens respectif, et dans lequel le découpage est effectué par entaillage dans les sens des au moins deux axes inclinés (C₂, C₃) de sorte que les sommets de filet de vis se faisant face dans le sens de la longueur soient alignés avec un décalage de sommet entre eux, dans lequel le décalage de sommet est sélectionné dans le groupe constitué de 1/3, 0,5, 1 et 1,5 sommets.

2. Plaque vissée (1) selon la revendication 1, dans lequel les axes (C₂, C₃) de la pluralité de trous de vis (5a-5f) disposés adjacents les uns aux autres dans le sens de la longueur sont situés à des positions décalées les unes par rapport aux autres ou dans des positions parallèles à des intervalles dans un sens perpendiculaire au sens de la longueur.

3. Plaque vissée (1) selon la revendication 1, comprenant en outre une pluralité de trous de vis (5a-5f) qui sont disposés adjacents les uns aux autres à des intervalles dans un sens croisant le sens de la longueur ou parallèlement au sens de la longueur, à l'extrémité dans le sens de la longueur.

4. Plaque vissée (1) selon la revendication 1, dans lequel le filetage intérieur (6) est un filetage intérieur conique.

5. Plaque vissée (1) selon la revendication 1, dans lequel le filetage intérieur (6) est formé dans une face intérieure du trou de vis (5a) qui a une forme sphérique.

6. Système de plaque vissée (2) comprenant:
une plaque vissée (1) selon l'une quelconque des revendications 1 à 5 ; et
une pluralité de vis (3) ou de corps de support sensiblement cylindriques destinés à fixer la plaque de vis (1) à un os (A),
dans lequel au moins l'un(e) des vis (3) ou des corps de support sensiblement cylindriques a, sur une face extérieure de sa tête, un filetage extérieur (3b) conçu pour être serré dans le filetage intérieur (6) d'un trou de vis correspondant (5a-5f) ménagé dans la plaque vissée (1).

7. Système de plaque vissée (2) comprenant:
une plaque vissée (1) selon la revendication 4; et
une pluralité de vis (3) ou de corps de support sensiblement cylindriques destiné(e)s à fixer la plaque de vis (1) à un os (A),
dans lequel au moins l'un(e) des vis (3) ou des corps de support sensiblement cylindriques a, sur une face extérieure de sa tête, un filetage extérieur conique (3b) conçu pour être serré dans le filetage intérieur conique (6) d'un trou de vis correspondant (5a-5f) ménagé dans la plaque vissée (1).

8. Système de plaque vissée (2) comprenant:
une plaque vissée (1) selon la revendication 5; et
une pluralité de vis (3) ou de corps de support sensiblement cylindriques destiné(e)s à fixer la plaque de vis (1) à un os (A),
dans lequel au moins l'un(e) des vis (3) ou des corps de support sensiblement cylindriques a, sur une face extérieure de sa tête sphérique, un filetage extérieur (3b) conçu pour être serré dans le filetage intérieur (6) sur une face intérieure d'un trou de vis sphérique correspondant (5a-5f) ménagé dans la plaque vissée (1).

9. Système de plaque vissée (2) comprenant:
une plaque vissée (1) selon l'une quelconque des revendications 1 à 5; et
une pluralité de vis (3) pour fixer la plaque de vis (1) à un os (A),
dans lequel au moins l'une des vis (3) est pourvue d'une tête qui a un filetage extérieur (3b) conçu pour être serré dans le filetage intérieur (6) d'un trou de vis correspondant (5a-5f) ménagé dans la plaque vissée (1) et une tige qui a un filetage extérieur (3a) dont le pas est égal à celui du filetage extérieur disposé sur la tête.

10. Système de plaque vissée (2) comprenant:
une plaque vissée (1) selon l'une quelconque des revendications 1 à 5; et
une pluralité de vis (3) pour fixer la plaque de vis (1) à un os (A),
dans lequel au moins l'une des vis (3) est pourvue d'une tête qui a un filetage extérieur (3a) conçu pour être serré dans le filetage intérieur (6) d'un trou de vis correspondant (5a-5f) ménagé dans la plaque vissée (1) et une tige qui a un filetage extérieur (3a) dont le pas est égal à celui du filetage extérieur disposé sur la tête.

11. Système de plaque vissée (2) comprenant:
une plaque vissée (1) selon l'une quelconque des revendications 1 à 5 ; et
une pluralité de vis (3) pour fixer la plaque de vis (1) à un os (A),
dans lequel au moins l'une des vis (3) est pourvue d'une tête qui a un filetage extérieur (3b) conçu pour être serré dans le filetage intérieur (6) d'un trou de vis correspondant (5a-5f) ménagé dans la plaque vissée (1) et une tige qui a un filetage extérieur (3a) dont le pas est supérieur à celui du filetage extérieur disposé sur la tête.

12. Système de plaque vissée (2) selon l'une quelconque des revendications 6 à 11, comprenant en outre une vis différente ayant une tête dont la dimension extérieure est supérieure à une dimension de diamètre intérieur d'un trou de vis correspondant et qui n'est pas doté d'un filetage extérieur sur sa face externe.

13. Système de plaque vissée (2) selon l'une quelconque des revendications 6 à 11,
dans lequel la plaque vissée (1) est dotée d'au moins un trou traversant ayant un contre-alésage dont la face de support est constituée d'une face interne sphérique ou une face interne cylindrique; et
le système de plaque vissée (2) comprend en outre une autre vis ayant une tête qui présente une forme complémentaire à celle de la surface de support du trou traversant et qui n'est pas dotée d'un filetage extérieur sur sa face externe.
